# FASCICULE DE BREVET EUROPEEN

(11) **EP 0 850 904 B1**
(45) Date de publication et mention de la délivrance du brevet: **05.06.2002**
(21) Numéro de dépôt: 97403049.6
(22) Date de dépôt: 15.12.1997
(51) Int. Cl.: C07C 11/09

(54) **Procédé de production d'isobutène de haute pureté combinant une distillation réactive d'hydroisomérisation, une distillation et une isomérisation squelettale**
Verfahren zur Herstellung von sehr reinem Isobuten, dass ein Reaktivdistillation Hydroisomerisierung, eine Distillation und eine Kohlenstoffgerüstisomerisierung kombiniert
Process for the preparation of very pure isobutene combining a reactive distillation hydroisomerisation, a distillation and a carbon skeleton isomerisation

(30) Priorité: 23.12.1996 FR 9616022
(43) Date de publication de la demande: 01.07.1998
(73) Titulaire: INSTITUT FRANCAIS DU PETROLE, 92852 Rueil-Malmaison Cedex (FR)
(72) Inventeur: Dorbon, Michel, 69002 Lyon (FR); Chodorge, Jean-Alain, 92160 Antony (FR); Cosyns, Jean, 78580 Maule (FR); Viltard, Jean-Charles, 26000 Valence (FR); Didillon, Blaise, 92500 Rueil Malmaison (FR)
(74) Mandataire: Andréeff, François

(56) Documents cités:
- FR-A- 2 130 387
- US-A- 4 104 321
- PATENT ABSTRACTS OF JAPAN vol. 10, no. 166 (C-353), 13 juin 1986 & JP 61 018732 A (MITSUI SEKIKU KAGAKU KOGYO), 27 janvier 1986,
- PATENT ABSTRACTS OF JAPAN vol. 13, no. 260 (C-607), 15 juin 1989 & JP 01 061434 A (TONEN SEKIYUKAGAKU), 8 mars 1989,

## Description

L'invention concerne un procédé de production d'isobutène de haute pureté à partir d'une coupe d'hydrocarbures comportant essentiellement des hydrocarbures oléfiniques ayant 4 atomes de carbone par molécule et comportant de l'isobutène, ainsi que du butène-1 et des butènes-2 en rapport correspondant sensiblement à l'équilibre thermodynamique, ledit procédé comprenant le passage de ladite coupe dans une zone de distillation associée à une zone réactionnelle d'hydroisomérisation, le fond de la zone de distillation comprenant des butènes-2 étant passé dans une seconde zone de distillation, de préférence extractive, permettant d'obtenir un effluent comprenant en majeure partie des butènes-2 qui est passé en majeure partie dans une zone d'isomérisation squelettale où les butènes linéaires sont au moins partiellement isomérisés en isobutène, l'effluent principal de la zone d'isomérisation squelettale étant au moins en partie recyclé en amont de la zone de distillation réactive.

L'isobutène destiné à la polymérisation doit avoir un niveau de pureté supérieur à 99 % et ne plus contenir que des traces de butène-1 et de butènes-2 (quelques dizaines de partie par million en poids, ppm). En effet, si le taux d'impureté dans l'isobutène est trop élevé, les polymères obtenus sont de moins bonne qualité et le rendement de la polymérisation est plus faible. Il est donc nécessaire d'éliminer d'une coupe d'hydrocarbures contenant de l'isobutène les autres hydrocarbures oléfiniques ayant 4 atomes de carbone par molécule. Le butène-1 et l'isobutène ayant des points d'ébullition très voisins, il n'est pas possible de les séparer par distillation à moins de mettre en oeuvre des moyens considérables. Les autres hydrocarbures oléfiniques ayant 4 atomes de carbone peuvent être séparés de l'isobutène par distillation.

Le principal problème qui se pose pour produire de l'isobutène haute pureté est donc la séparation du butène-1 de l'isobutène. Pour effectuer cette séparation plusieurs voies sont envisageables.

La première voie consiste en une extraction à l'acide sulfurique : l'isobutène est hydraté sélectivement et régénéré ensuite par traitement de la phase aqueuse. Si la température et la concentration sont bien contrôlés, ce procédé permet d'obtenir de l'isobutène de bonne pureté. Cependant, le rendement n'excède habituellement pas 90 %, l'extraction n'étant pas complète et il se forme des dimères et oligomères, conduisant à la formation de boues acides toxiques.

La deuxième voie consiste en un craquage de l'éther méthylique de l'alcool tertio-butylique (MTBE) : l'isobutène est extrait de la coupe C₄ en le faisant réagir avec du méthanol afin de former du MTBE. Le MTBE est alors craqué en méthanol et isobutène sur un catalyseur acide. Le rendement de récupération peut-être d'au moins 96 %. L'isobutène produit est de bonne pureté mais il doit être débarrassé du diméthyléther qui peut se former durant le craquage.

La troisième voie envisageable est la déshydratation de l'alcool butylique tertiaire (ABT). Dans l'opération précédente, le méthanol peut être remplacé par l'eau, ce qui conduit à la production d'ABT. L'isobutène est ensuite récupéré par déshydratation de l'ABT. Cette voie n'est pratiquement pas utilisée, essentiellement parce que l'ABT est très lié au marché de l'oxyde de propylène. L'ABT peut, selon les procédés, être un sous-produit de l'oxyde de propylène.

Le problème de la séparation butène-1 - isobutène a été posé depuis longtemps. Diverses solutions ont été apportées donc aucune n'est totalement satisfaisante en termes d'efficacité de séparation sans apparition de produit(s) parasite(s). Ainsi le brevet US-A-2403672 décrit un procédé de séparation d'isobutène à partir d'un mélange isobutène-butène-1 qui comprend l'introduction du mélange dans une zone d'isomérisation et de fractionnement dans laquelle le catalyseur d'isomérisation joue également le rôle de corps de garnissage assurant la fonction de distillation. Cette solution présente l'inconvénient majeur de ne pas avoir une bonne efficacité de distillation et donc une médiocre capacité de séparation de l'isobutène du butène-1.

La demande de brevet FR-A-2 130 387 décrit un procédé d'isomérisation d'oléfines visant à séparer l'isobutène d'un mélange en C₄ contenant du butène-1. Ce procédé comprend l'isomérisation du mlange en C₄, la séparation du mélange isomérisé en une fraction de tête contenant de l'isobutène et le restant de butène-1 et en une fraction de queue contenant les butènes-2, l'isomérisation de ladite fraction de tête, la réintroduction avec le mélange isomérisé, dans la zone de séparation, d'une partie de la fraction de tête isomérisée résultante et la purification ultérieure de la partie restante de la fraction de tête isomérisée par fractionnement. Ce procédé antérieur réalise la purification de l'isobutène à partir de la fraction de tête de la zone de séparation en la soumettant à au moins un autre fractionnement, sans s'intéresser à la valorisation de l'effluent riche en butènes-2.

Le procédé selon l'invention permet de produire de l'isobutène de haute pureté, au moindre coût et avec un excellent rendement, à partir d'une coupe C₄ oléfinique contenant au moins de l'isobutène, ainsi que du butène-1 et des butènes-2 en rapport correspondant sensiblement à l'équilibre thermodynamique, généralement issue d'un procédé de vapocraquage, telle que la coupe C4 brute ou le raffinat-1 (obtenu après extraction du butadiène de la coupe C4 brute), ou de craquage catalytique. Le procédé selon l'invention est caractérisé par l'intégration d'opérations de distillation, d'hydroisomérisation et d'isomérisation squelettale des butènes linéaires en isobutène, agencées et opérées de manière à minimiser le coût d'investissement du procédé, à maximiser la conversion du butène-1 en butènes-2, à minimiser l'hydrogénation de l'isobutène en isobutane et à transformer une partie des butènes linéaires en isobutène afin de maximiser le rendement en isobutène. Ainsi, le procédé selon l'invention peut réaliser au moins partiellement l'hydrogénation sélective des composés polyinsaturés le plus souvent dièniques ou acétyléniques tels que le butadiène, le vinylacétylène, le méthylacétylène et l'éthylacétylène, quand ces composés sont présents dans la charge, l'hydroisomérisation du butène-1 en butènes-2 (cis et trans), et l'isomérisation squelettale de butènes linéaires en isobutène. Les butènes-2 produits de cette hydrogénation et de cette hydroisomérisation peuvent alors être séparés de l'isobutène par distillation, ce qui n'est pas le cas du butène-1. Le fond de la zone de la distillation réactive, qui contient principalement des butènes-2 et généralement du normal butane, passe dans une seconde zone de distillation, de préférence extractive, afin de le débarraser au mieux des impuretés que sont les alcanes, puis dans une unité d'isomérisation squelettale où les butènes linéaires sont, au moins partiellement, transformés en isobutène ; les produits de cette isomérisation squelettale sont recyclés en amont de la distillation réactive, par exemple en mélange avec la charge de la zone de distillation réactive, afin de séparer l'isobutène produit des butènes linéaires. Par rapport aux autres procédés cités précédemment, le procédé selon l'invention a l'avantage d'avoir un rendement en isobutène très élevé et généralement toujours supérieur à 100 % si des butènes linéaires sont présents dans la charge. Dans le cas où la charge est une coupe C4 issue d'une unité de vapocraquage dont la majeure partie du butadiène a été extrait, c'est-à-dire un raffinat-1, les rendement sont généralement supérieurs à 120 %.

Le procédé selon l'invention est un procédé de traitement d'une charge, comprenant en majeure partie des hydrocarbures oléfiniques comportant 4 atomes de carbone par molécule dont de l'isobutène, ainsi que du butène-1 et des butènes-2 en rapport correspondant sensiblement à l'équilibre thermodynamique, dans lequel on traite ladite charge dans une zone de distillation, comportant généralement une zone d'épuisement et une zone de rectification, associée à une zone réactionnelle d'hydroisomérisation, de manière à assurer la continuité de la distillation, et de façon à sortir finalement en tête de la zone de distillation un effluent riche en isobutène, généralement de haute pureté, et en fond de zone de distillation un effluent riche en butènes-2, dont la majeure partie est envoyée dans une seconde zone de distillation, de préférence extractive, qui permet d'obtenir un premier effluent comprenant en majeure partie des butènes-2 et un second effluent comprenant en majeure partie du normal butane, ledit premier effluent étant passé en majeure partie dans une zone d'isomérisation squelettale et l'effluent de la zone d'isomérisation squelettale étant recyclé en amont de la zone de distillation (par rapport au sens général de circulation d'effluent dans le procédé). Ledit procédé permet la production d'isobutène de haute pureté.

La charge qui alimente la zone de distillation est introduite dans ladite zone généralement au moins à un niveau de ladite zone, de préférence principalement à un seul niveau de ladite zone. Elle est en rapport correspondant sensiblement à l'équilibre thermodynamique butène-1 - butènes-2 à l'introduction. Une des mises en oeuvre préférées du procédé selon l'invention comprend l'obtention de ladite charge à partir d'une coupe comprenant en majeure partie des hydrocarbures oléfiniques comportant 4 atomes de carbone par molécule, par traitement de ladite coupe dans une première zone d'hydroisomérisation, généralement indépendante de la partie externe éventuelle de la zone réactionnelle d'hydroisomérisation associée à la zone de distillation, la majeure partie de l'effluent de ladite première zone d'hydroisomérisation servant alors de charge, principale ou secondaire selon les définitions données ci-après dans le texte, qui alimente la zone de distillation. Dans un tel cas le recyclage d'une partie, de préférence de la majeure partie, de l'effluent de la zone d'isomérisation squelettale en amont de la zone de distillation est de préférence réalisé en amont de la première zone d'hydroisomérisation. Si ladite charge comprend des composés polyinsaturés, le plus souvent diéniques et/ou acétyléniques, lesdits composés sont de préférence transformés en butènes par la première zone d'hydroisomérisation avant l'introduction dans la zone de distillation.

Toute autre technique permettant d'obtenir, à partir d'une coupe comprenant en majeure partie des hydrocarbures oléfiniques en C₄ dont du butène-1 et de l'isobutène, une charge où le butène-1 et les butènes-2 sont en rapport correspondant sensiblement à l'équilibre thermodynamique est aussi envisageable dans le cadre de l'invention.

La première zone réactionnelle d'hydroisomérisation éventuelle, située en amont de la zone de distillation-réaction, réalise au moins partiellement l'hydrogénation sélective des composés polyinsaturés, le plus souvent diéniques tel que le butadiène, en plus de l'hydroisomérisation d'au moins une partie du butène-1 en butènes-2. Elle comprend généralement au moins un lit catalytique d'hydroisomérisation comprenant un catalyseur d'hydroisomérisation, de préférence de 1 à 4 lit(s) catalytique(s) dans le cas où au moins deux lits catalytiques se trouvent incorporés dans ladite zone réactionnelle, ces deux lits sont de préférence répartis dans au moins deux réacteurs, répartis en série ou en parallèle, de préférence en série. Par exemple ladite première zone réactionnelle comprend un seul réacteur dans lequel se trouve au moins un, et de préférence un seul, lit catalytique. Une des mises en oeuvre préférées du procédé de la présente invention est telle que ladite première zone réactionnelle comprend deux réacteurs généralement répartis en série comportant chacun au moins un, de préférence un seul, lit catalytique. Lorsque ladite zone réactionnelle comprend au moins deux réacteurs, le recyclage éventuel d'au moins une partie de l'effluent d'au moins un des réacteurs compris dans la première zone réactionnelle dans ladite première zone est effectué généralement à l'entrée d'un réacteur, de préférence dudit réacteur, de préférence avant l'injection du composé gazeux comprenant de l'hydrogène. Il est aussi possible de procéder à un recyclage autour de ladite première zone elle-même, c'est-à-dire généralement à l'entrée du premier réacteur de ladite zone, de préférence avant l'injection du composé gazeux comprenant de l'hydrogène ; par exemple, dans le cas de deux réacteurs, au moins une partie de l'effluent du second réacteur est recyclée vers l'entrée du premier réacteur. Ceci permet avantageusement d'abaisser la teneur en composés polyinsaturés dans l'effluent de ladite première zone réactionnelle.

Les conditions opératoires de la première zone d'hydroisomérisation, lorsqu'elle est présente, sont généralement les suivantes : le catalyseur est identique au catalyseur de la zone d'hydroisomérisation qui sera décrit ci-après. La pression est généralement comprise entre 4 et 40 bar (1 bar = 0,1 MPa), de préférence entre 6 et 30 bar. La température est généralement comprise entre 10 et 150°C, de préférence entre 20 et 100°C. Le rapport molaire H₂/hydrocarbures est généralement ajusté de façon à obtenir une conversion pratiquement totale des composés polyinsaturés tel que le butadiène et une isomérisation suffisante du butène-1 en butènes-2 avec formation limitée d'alcanes.

La zone réactionnelle d'hydroisomérisation associée à la zone de distillation comprend généralement au moins un lit catalytique d'hydroisomérisation comprenant un catalyseur d'hydroisomérisation, de préférence de 2 à 6, et de façon encore plus préférée de 2 à 4 lits catalytiques ; dans le cas où au moins deux lits catalytiques se trouvent incorporés dans ladite zone de distillation, ces deux lits sont de préférence séparés par au moins un interne de distillation. Ladite zone réactionnelle d'hydroisomérisation réalise au moins partiellement l'hydroisomérisation d'au moins une partie, de préférence la majeure partie, du butène-1 présent dans sa charge en butènes-2 (cis et trans), généralement de telle façon que la teneur en butène-1 de l'effluent de tête de la zone de distillation soit au maximum égale à une certaine teneur.

La zone réactionnelle d'hydroisomérisation associée à la zone de distillation réalise au moins partiellement l'hydroisomérisation du butène-1 présent dans la phase liquide (reflux) coulant à la hauteur du niveau de prélèvement de la zone de distillation. Elle est soit totalement interne à ladite zone de distillation, soit totalement externe à ladite zone de distillation, soit à la fois interne et externe à ladite zone de distillation.

Un des mises en oeuvre préférées du procédé selon l'invention comprend l'alimentation de la zone de distillation, en plus de l'alimentation en la charge principale, en une charge dite secondaire (par rapport à la charge principale), qui provient ou non d'une zone réactionnelle d'hydroisomérisation telle que la première zone réactionnelle éventuelle d'hydroisomérisation, indépendamment ou non de l'alimentation de la zone de distillation en la charge principale. La charge secondaire est généralement une coupe C4 contenant au moins de l'isobutène, ainsi que du butène-1 et des butènes-2 en rapport correspondant sensiblement à l'équilibre thermodynamique, et est généralement issue d'un procédé de vapocraquage, telle que la coupe C4 brute ou le raffinat-1, ou de craquage catalytique ; généralement et de façon préférée, la charge secondaire est une coupe C4 essentiellement exempte de composés polyinsaturés et sa teneur en butène-1 est inférieure à la teneur en butène-1 de la charge principale. Si la teneur en composés insaturés de la charge secondaire est élevée, ladite charge est de préférence traitée dans une zone d'hydrogénation sélective avant son entrée dans la zone de distillation.

Lorsque la charge principale est introduite en un seul niveau d'introduction, la charge secondaire est généralement introduite dans la zone de distillation en au moins un niveau d'introduction, de préférence en un seul niveau d'introduction, ledit niveau d'introduction dépendant de la composition de ladite charge secondaire. Ainsi, dans un premier exemple la charge secondaire peut être très riche en isobutène et contenir moins de 1,5 fois de butène-1 que la charge principale n'en contient, auquel cas la charge secondaire est de préférence introduite en un seul niveau situé généralement au-dessus du niveau d'introduction de la charge principale. Ou bien dans un second exemple la charge secondaire peut être pratiquement exempte de butène-1, auquel cas la charge secondaire est de préférence introduite en un seul niveau situé généralement au-dessous du niveau d'introduction de la charge principale. Il est aussi possible de procéder au mélange éventuel de la charge principale, avant son entrée en zone de distillation, et de la charge secondaire.

La zone de distillation comprend généralement au moins une colonne munie d'au moins un interne de distillation choisi dans le groupe formé par les plateaux simples, les plateaux à multi-déversoirs, les garnissages en vrac et les garnissages structurés, ainsi qu'il est connu de l'homme du métier, de telle sorte que l'efficacité globale totale est au moins égale à cinq étages théoriques. Dans les cas connus de l'homme du métier où la mise en oeuvre d'une seule colonne pose des problèmes, on préfère généralement scinder ladite zone de façon à utiliser finalement au moins deux colonnes qui, mises bout à bout, réalisent ladite zone, c'est-à-dire que la zone de rectification, la zone réactionnelle éventuelle et la zone d'épuisement se répartissent sur les colonnes. En pratique, lorsque la zone réactionnelle est au moins en partie interne à la zone de distillation, la zone de rectification ou la zone d'épuisement, et de préférence la zone d'épuisement, peut généralement se trouver dans au moins une colonne différente de la colonne comprenant la partie interne de la zone réactionnelle.

Une première mise en oeuvre du procédé selon l'invention est telle que la zone réactionnelle d'hydroisomérisation associée à la zone de distillation est au moins en partie externe à ladite zone de distillation. Généralement, le procédé selon l'invention comprend de 1 à 6, de préférence de 2 à 4 niveau(x) de prélèvement qui alimente(nt) la partie externe de ladite zone d'hydroisomérisation. Dans un tel cas, le liquide à hydroisomériser, soit partiellement, soit totalement, circule d'abord dans la partie externe de la zone d'hydroisomérisation puis dans la partie interne de ladite zone. Alors, deux cas peuvent se présenter. Dans le premier cas, la partie externe de la zone d'hydroisomérisation est alimentée par un seul niveau de prélèvement, et alors, si ladite partie comprend plus de deux réacteurs, ceux-ci sont disposés en série ou en parallèle. Dans le second cas, préféré selon la présente invention, la partie externe de la zone d'hydroisomérisation est alimentée par au moins deux niveaux de prélèvement. Une partie de la partie externe de ladite zone d'hydroisomérisation qui est alimentée par un niveau de prélèvement donné, si ladite partie externe comprend au moins deux niveaux de prélèvement, comprend généralement au moins un réacteur, de préférence un seul réacteur. Si ladite partie de la partie externe comprend au moins deux réacteurs, chaque réacteur externe à la zone de distillation est alimenté par un seul niveau de prélèvement, de préférence associé à un seul niveau de réintroduction, ledit niveau de prélèvement étant distinct du niveau de prélèvement qui alimente le(s) autre(s) réacteur(s).

Le procédé selon l'invention est généralement tel que la charge de toute partie de la zone réactionnelle d'hydroisomérisation, qu'elle soit interne ou éventuellement externe, est prélevée à la hauteur d'un niveau de prélèvement et représente au moins une partie, de préférence la majeure partie, du liquide (reflux) coulant dans la zone de distillation, de préférence coulant dans la zone de rectification et de façon encore plus préférée coulant à un niveau intermédiaire de la zone de rectification, l'effluent de la zone réactionnelle d'hydroisomérisation étant au moins en partie, de préférence en majeure partie, réintroduit dans la zone de distillation, de manière à assurer la continuité de la distillation.

Dans le cas de ladite première mise en oeuvre, le procédé selon l'invention permet d'isomériser une grande partie du butène-1 en butènes-2 à l'extérieur de la zone de distillation, éventuellement dans des conditions de pression et/ou de température différentes de celles utilisées dans la colonne. De préférence, la température à l'entrée (respectivement à la sortie) du niveau de prélèvement qui alimente un lit catalytique de la partie de la zone d'hydroisomérisation située à l'extérieur de la colonne, est sensiblement semblable, c'est-à-dire que l'écart est sensiblement inférieur à 10°C par rapport à la température à la hauteur du niveau de prélèvement (respectivement du niveau de réintroduction). De même, on peut avantageusement effectuer la réaction d'hydroisomérisation dans la partie de la zone réactionnelle située à l'extérieur de la colonne à une pression plus élevée que celle utilisée à l'intérieur de la zone de distillation. Cette augmentation de pression permet aussi une dissolution accrue du flux gazeux contenant de l'hydrogène dans la phase liquide contenant le butène-1 à isomériser.

Dans la partie de la zone d'hydroisomérisation associée à la zone de distillation externe à ladite zone de distillation, les conditions opératoires sont généralement indépendantes des conditions opératoires de la zone de distillation. Elles sont généralement les suivantes. La pression requise pour cette étape d'hydroisomérisation est généralement d'environ 1 à 40 bar absolus, de préférence d'environ 2 à 30 bar, et de façon encore plus préférée d'environ 4 à 25 bar. La température opératoire de ladite zone d'hydroisomérisation est généralement d'environ 20 à 150°C, de préférence d'environ 40 à 100°C, et de façon préférée d'environ 40 à 80°C. La vitesse spatiale au sein de ladite zone d'hydroisomérisation, calculée par rapport au catalyseur, est généralement d'environ 1 à 100 et plus particulièrement d'environ 4 à 50 h⁻¹ (volume de charge par volume de catalyseur et par heure). Le débit d'hydrogène correspondant est tel que le rapport molaire H₂/hydrocarbures entrant dans ladite zone d'hydroisomérisation est de préférence au moins égal à 10⁻⁵. Ce rapport est le plus souvent d'environ 10⁻⁵ à environ 3 et très fréquemment d'environ 10⁻⁴ à environ 1. Le catalyseur est, dans ladite partie de la zone d'hydroisomérisation externe à la zone de distillation, disposé suivant toute technologie connue de l'homme du métier.

Pour la réalisation de l'hydroisomérisation selon le procédé de l'invention, le rapport molaire théorique d'hydrogène nécessaire pour la conversion désirée du butène-1 est tel que le rapport molaire H₂/hydrocarbures entrant dans la zone réactionnelle asociée à la zone de distillation est au moins égal à 10⁻⁵. Ce rapport molaire peut-être optimisé de telle manière que d'une part tout l'hydrogène soit consommé dans les réactions d'hydroisomérisation afin d'éviter un dispositif de récupération de l'hydrogène en sortie de la zone réactionnelle, d'autre part de minimiser les réactions parasites d'hydrogénation de l'isobutène afin de maximiser le rendement du procédé en isobutène et enfin de telle manière qu'il y a suffisamment d'hydrogène tout le long de la zone réactionnelle pour que la réaction d'hydroisomérisation du butène-1 en butènes-2 puisse se faire. Cependant si les conditions sont telles qu'il y ait excès d'hydrogène, l'hydrogène en excès peut être avantageusement récupéré par exemple selon l'une des techniques décrites ci-après. Par exemple l'hydrogène en excès qui sort en tête de zone de distillation est récupéré, puis injecté en amont des étapes de compression associées à une unité de réformage catalytique, en mélange avec de l'hydrogène provenant de ladite unité, ladite unité opérant de préférence à basse pression (soit généralement une pression inférieure à 8 bar). Cet hydrogène en excès peut aussi être récupéré, puis comprimé et réutilisé dans ladite zone réactionnelle.

Dans le cas de ladite première mise en oeuvre, le procédé selon l'invention comprend l'utilisation de la technique dite de "pump-around", c'est-à-dire de pompage en boucle, qui consiste à faire passer à l'extérieur de la zone de distillation une partie, de préférence la majeure partie, du liquide (reflux) par un facteur de préférence compris entre 0,5 et 1,5, de manière plus préférée compris entre 0,75 et 1,3, c'est-à-dire que le débit d'un lit catalytique de la partie externe de la zone d'hydroisomérisation associée à la zone de distillation, ledit lit étant alimenté à un niveau de prélèvement par au moins une partie de l'effluent liquide (reflux) coulant sur le plateau de distillation associé audit niveau de prélèvement (i.e. sur lequel on prélève ladite partie d'effluent liquide) et par au moins une partie du liquide correspondant au recyclage de l'effluent dudit lit sensiblement au-dessus ou sensiblement au-dessous ou sensiblement à la même hauteur que ledit niveau de prélèvement, est inférieur ou supérieur à 1 fois le débit de liquide coulant sur ledit plateau, de manière encore plus préférée égal à 1 fois le débit de liquide coulant sur ledit plateau, soit généralement du même ordre de grandeur que le débit de liquide coulant sur ledit plateau.

Selon un des modes de réalisation de ladite première mise en oeuvre du procédé selon l'invention, la zone d'hydroisomérisation est à la fois partiellement incorporée dans la zone de distillation, c'est-à-dire interne à la zone de distillation, et partiellement externe à la zone de distillation. Selon un tel mode de réalisation, la zone d'hydroisomérisation comprend au moins deux, de préférence au moins trois lits catalytiques, au moins un lit catalytique étant interne à la zone de distillation, et au moins un lit catalytique étant externe à la zone de distillation. Pour la partie éventuelle de la zone réactionnelle externe à la zone de distillation, la réintroduction de l'effluent en zone de distillation se fait sensiblement à proximité, c'est-à-dire généralement sensiblement à la même hauteur ou sensiblement au-dessus ou sensiblement au-dessous, le plus souvent sensiblement à la même hauteur ou sensiblement au-dessus, c'est-à-dire situé à une distance correspondant à une hauteur comprise entre 0 et 4 plateaux théoriques d'un niveau de prélèvement, de préférence dudit niveau de prélèvement, ceci pour assurer la continuité de la distillation. Pour la partie de la zone réactionnelle interne à la zone de distillation, le prélèvement de liquide (reflux) est fait naturellement par écoulement dans la partie de la zone réactionnelle interne à la zone de distillation, et la réintroduction de liquide en zone de distillation se fait aussi naturellement par écoulement du liquide à partir de la partie de la zone réactionnelle interne à la zone de distillation. De plus, le procédé selon l'invention est de préférence tel que l'écoulement du liquide contenant le réactif, le butène-1, est co-courant ou contre-courant à l'écoulement du flux gazeux comprenant de l'hydrogène, pour tout lit catalytique de la partie interne de la zone d'hydroisomérisation contenue dans la zone de distillation.

Selon un autre des modes de réalisation préférés de la première mise en oeuvre du procédé selon l'invention, indépendant du mode de réalisation précédent, la zone d'hydroisomérisation est en totalité externe à la zone de distillation. Elle a alors les caractéristiques de la partie externe de la zone d'hydroisomérisation du mode de réalisation précédent.

L'hydrogène, compris dans le flux gazeux, utilisé dans le procédé de l'invention pour l'hydroisomérisation du butène-1 en butènes-2, que ce soit dans la première zone d'hydroisomérisation éventuelle ou bien dans la zone d'hydroisomérisation associée à la zone de distillation, provient généralemnt en majeure partie, de préférence en quasi totalité, de l'extérieur de la zone de distillation. Il peut provenir de toute source produisant de l'hydrogène à au moins 50% volume de pureté, de préférence au moins 80% volume de pureté, et de façon encore plus préférée au moins 90% volume de pureté. Par exemple, on peut citer l'hydrogène provenant des procédés de vapocraquage, de réformage catalytique, de P.S.A. (adsorption par alternance de pression) ou de génération électrochimique.

Lorsque la zone d'hydroisomérisation associée à la zone de distillation est au moins en partie incorporée à ladite zone de distillation, le catalyseur d'hydroisomérisation peut être disposé dans ladite partie incorporée suivant les différentes technologies proposées pour conduire des distillations catalytiques. Elles sont essentiellement de deux types.

Suivant le premier type de technologies, la réaction et la distillation procèdent simultanément dans le même espace physique, comme l'enseignent par exemple la demande de brevet WO-A-90/02.603, les brevets US-A-4.471.154, US-A-4.475.005, US-A-4.215.011, US-A-4.307.254, US-A-4.336.407, US-A-4.439.350, US-A-5.189.001, US-A-5.266.546, US-A-5.073.236, US-A-5.215.011, US-A-5.275.790, US-A-5.338.517, US-A-5.308.592, US-A-5.236.663, US-A-5.338.518, ainsi que les brevets EP-B1-0.008.860, EP-B1-0.448.884, EP-B1-0.396.650 et EP-B1-0.494.550 et la demande de brevet EP-A1-0.559.511. Le catalyseur est alors généralement en contact avec une phase liquide descendante, générée par le reflux introduit au sommet de la zone de distillation, et avec une phase vapeur ascendante, générée par la vapeur de rebouillage introduite en fond de zone. Selon ce type de technologies, le flux gazeux comprenant de l'hydrogène nécessaire à la zone réactionnelle, pour la réalisation du procédé selon l'invention, pourrait être joint à la phase vapeur, de préférence sensiblement à l'entrée d'au moins un lit catalytique de la zone réactionnelle.

Suivant le second type de technologies, le catalyseur est disposé de telle façon que la réaction et la distillation procèdent généralement de manière indépendante et consécutive, comme l'enseignent par exemple les brevets US-A-4.847.430, US-A-5.130.102 et US-A-5.368.691, la vapeur de la distillation ne traversant pratiquement pas tout lit catalytique de la zone réactionnelle. Ainsi, si l'on utilise ce type de technologie, le procédé selon l'invention est généralement tel que l'écoulement du liquide à hydroisomériser est co-courant à l'écoulement du flux gazeux comprenant de l'hydrogène et tel que la vapeur de distillation n'est pratiquement pas en contact avec le catalyseur (ce qui se traduit généralement en pratique par le fait que ladite vapeur est séparée dudit liquide à hydroisomériser), pour tout lit catalytique de la partie interne de la zone d'hydroisomérisation. De tels systèmes comportent généralement au moins un dispositif de distribution de liquide qui peut être par exemple un répartiteur de liquide, dans tout lit catalytique de la zone réactionnelle. Néanmoins, dans la mesure où ces technologies ont été conçues pour des réactions catalytiques intervenant entre des réactifs liquides, elles ne peuvent convenir sans modification pour une réaction catalytique d'hydroisomérisation, pour laquelle l'un des réactifs, l'hydrogène, est à l'état gazeux.

Pour tout lit catalytique de la partie interne de la zone d'hydroisomérisation, il est donc généralement nécessaire d'adjoindre un dispositif d'introduction du flux gazeux comprenant de l'hydrogène, par exemple selon les techniques décrites ci-après. Ainsi, la partie interne de la zone d'hydroisomérisation comporte au moins un dispositif de distribution de liquide et au moins un dispositif d'introduction du flux gazeux dans tout lit catalytique de la partie interne de la zone d'hydroisomérisation. Selon une première technique, le dispositif d'introduction du flux gazeux dans tout lit catalytique est identique au dispositif de distribution de liquide dans le lit catalytique, c'est-à-dire qu'il existe un moyen d'introduction du gaz dans le liquide en mont du dispositif de distribution de liquide (par rapport au sens de circulation du liquide). En pratique en en langage couratn, ceci revient à réaliser un piquage du gaz dans le liquide en mont du moyen de distribution de liquide. Selon une autre technique, le dispositif d'introduction du flux gazeux est disposé sensiblement au niveau du dispositif de distribution de liquide, au dessous ou au sein, de préférence non loin du dispositif de distribution de liquide, du lit catalytique, c'est-à-dire que le gaz et le liquide sont introduits de façon séparée dans le lit catalytique.

Ainsi, lorsque la zone d'hydroisomérisation associée à la zone de distillation est au moins en partie interne à ladite zone de distillation, une des réalisations préférées du procédé selon l'invention est telle que le catalyseur de la partie interne de ladite zone d'hydroisomérisation est disposé dans ladite partie suivant le dispositif de base décrit dans le brevet US-A-5.368.691, aménagé de manière que tout lit catalytique de la partie interne de la zone d'hydroisomérisation soit alimenté par un flux gazeux comprenant de l'hydrogène, régulièrement distribué à sa base, par exemple selon l'une des trois techniques décrites ci-dessus. Suivant cette technologie, dans laquelle la zone de distillation comprend une seule colonne et dans laquelle la zone d'hydroisomérisation est en totalité interne à ladite colonne (ce qui diffère du procédé selon l'invention), le catalyseur compris dans tout lit catalytique, interne à la zone de distillation, est alors en contact avec une phase liquide ascendante, générée par le reflux introduit au sommet de la colonne de distillation, et avec le flux gazeux comprenant de l'hydrogène qui circule dans le même sens que le liquide; le contact avec la phase vapeur de la distillation étant évité en faisant transiter cette dernière par au moins une cheminée spécialement aménagée.

Lorsque la zone d'hydroisomérisation associée à la zone de distillation est au moins en partie interne à ladite zone de distillation, les conditions opératoires de ladite partie interne sont liées aux conditions opératoires de la distillation. La distillation est généralement conduite de manière à minimiser la quantité d'isobutène dans le produit de fond afin de maximiser le rendement du procédé en isobutène et de manière à minimiser la quantité de butènes-2 et butène-1 dans le produit de tête, afin d'avoir en tête de l'isobutène de haute pureté. Elle est réalisée sous une pression généralement comprise entre 2 et 30 bar, de préférence entre 4 et 15 bar, de façon encore plus préférée entre 4 et 10 bar (1 bar = 10⁵ Pa), avec un taux de reflux compris entre 1 et 30, et de préférence compris entre 5 et 20. La température de tête de zone de distillation est comprise généralement entre 0 et 200 °C et la température de fond de zone de distillation est comprise généralement entre 5 et 250 °C. Les températures de la zone de distillation peuvent être calculées à partir des pressions, ainsi qu'il est connu de l'homme du métier. La réaction d'hydroisomérisation est conduite dans des conditions qui sont le plus généralement intermédiaires entre celles établies en tête et en fond de zone de distillation, à une température comprise entre 20 et 150 °C, et de préférence comprise entre 40 et 80 °C, et à une pression comprise entre 2 et 30 bar, de préférence entre 4 et 15 bar, de façon encore plus préférée entre 4 et 10 bar. Le liquide soumis à l'hydroisomérisation est alimenté par un flux gazeux comprenant, de préférence en majeure partie, de l'hydrogène.

Une seconde mise en oeuvre du procédé selon l'invention est telle que la zone réactionnelle d'hydroisomérisation est en totalité interne à la zone de distillation.

De façon plus générale, le catalyseur utilisé dans toute zone d'hydroisomérisation selon le procédé de la présente invention comprend généralement au moins un métal choisi dans le groupe formé par les métaux nobles du groupe VIII de la Classification Périodique des éléments et le nickel, c'est-à-dire choisi dans le groupe formé par le ruthénium, le rhodium, le palladium, l'osmium, l'iridium, le platine, de préférence le palladium, ou le nickel, utilisé tel quel ou de préférence déposé sur un support. Le métal doit généralement se trouver sous forme réduite au moins pour 50 % en poids de sa totalité. La teneur en métal noble du catalyseur est habituellement d'environ 0,01 à environ 2 % en poids. Dans le cas de l'utilisation de nickel, la proportion de nickel par rapport au poids total de catalyseur est comprise entre 5 et 70%, et de façon préférée entre 10 et 70 %, et on utilise généralement un catalyseur tel que la taille moyenne des cristallites de nickel est inférieure à 10 nm, de préférence inférieure à 8 nm, de façon encore plus préférée inférieure à 6 nm. Mais tout autre catalyseur d'hydroisomérisation connu de l'homme du métier peut également être choisi. Le catalyseur est habituellement traité par un composé du soufre puis par de l'hydrogène avant son utilisation. Le catalyseur est généralement sulfuré in situ ou ex situ de telle façon que du soufre soit chimisorbé sur une partie au moins du métal. Le soufre chimisorbé a pour effet de favoriser la réaction d'hydroisomérisation du butène-1 en butènes-2 par rapport à la réaction d'hydrogénation de l'isobutène et donc de maximiser le rendement en isobutène du procédé.

Le support du catalyseur d'hydroisomérisation est généralement choisi dans le groupe formé par l'alumine, les silice-alumines, la silice, les zéolithes, le charbon actif, les argiles, les ciments alumineux, les oxydes de terres rares et les oxydes alcalino-terreux, seuls ou en mélange. On utilise de préférence un support à base d'alumine ou de silice, de surface spécifique comprise entre 10 et 300 m²/g, de préférence entre 30 et 70 m²/g.

A titre d'exemple non limitatif utilisable dans le cadre de la présente invention on peut citer les catalyseurs commerciaux tel que celui vendu par la société Catalysts and Chemicals sous la référence C-31, celui vendu par la société Girdler Corporation sous la référence G-55 ou de préférence ceux vendus par la société Procatalyse sous la référence LD-265, LD-265S, LD-267 et LD-267R.

L'effluent de fond de la zone de distillation qui contient généralement principalement des butènes-2 et le plus souvent du normal butane est traité en majeure partie dans une zone de distillation, de préférence extractive. Le normal butane qui est généralement dans ledit effluent provient principalement de la charge du procédé selon l'invention et éventuellement est un peu généré dans les zones d'hydroisomérisation par hydrogénation des butènes. Ladite zone de distillation permet l'obtention d'un premier effluent, de fond dans le cas d'une distillation extractive, comprenant en majeure partie des butènes-2 et pratiquement pas de normal butane, et d'un second effluent comprenant en majeure partie du normal butane et pratiquement pas de butènes-2. Le premier effluent est en majeure partie envoyé en tant que charge de la zone d'isomérisation squelettale du procédé. Le fait de rajouter un moyen de séparation des butènes-2 et du normal butane en amont de la zone d'isomérisation squelettale du procédé, lorsque la charge contient du normal butane, permet de diminuer fortement la quantité de normal butane qui est passée dans la zone d'isomérisation squelettale du procédé ; cela permet donc de diminuer la capacité de la zone d'isomérisation squelettale du procédé selon l'invention. L'avantage d'utiliser une distillation extractive par rapport à tout autre moyen de séparation de ces deux composés tel qu'une distillation classique tient au fait que la différence entre les points d'ébullition du normal butane et des butènes-2 est faible et donc, pour une même séparation, une distillation normale nécessiterait beaucoup plus de plateaux théorisques et donc un coût plus important.

La zone de seconde de distillation est dans une mise en oeuvre préférée du procédé selon l'invention une zone de distillation extractive. Une telle zone de distillation extractive est réalisée de façon appropriée en présence d'un solvant non aqueux sélectif des hydrocarbures oléfiniques qui est introduit au moins en partie dans une première partie de la zone de distillation extractive, permettant de récupérer une fraction de tête riche en normal butane, constituant en majeure partie ledit second effluent de la zone de distillation extractive et une fraction de queue que l'on traite dans la seconde partie de la zone de distillation extractive, permettant de récupérer en tête au moins en partie un distillat riche en hydrocarbures oléfiniques, qui constitue principalent ledit premier effluent de la zone de distillation extractive, et en queue un résidu contenant essentiellement du solvant. Le solvant mis en jeu est généralement un solvant ayant une substantielle sélectivité vis-à-vis des oléfines. En particulier il peut être choisi parmi les solvants dans le groupe formé par la N-N diméthylformamide, la N-N diéthylformamide, la N-N diméthylacétamide, la formylmorpholine, la N-méthylpyrrolidone, la butyrolactone et le furfural.

Dans la zone d'isomérisation squelettale du procédé selon l'invention, les butènes linéaires présents dans le premier effluent de la seconde zone de distillation sont en partie, de préférence en majeure partie, isomérisés en isobutène. L'effluent de la zone d'isomérisation squelettale du procédé selon l'invention contient alors de l'isobutène formé lors de la réaction et des butènes linéaires. L'effluent de la zone d'isomérisation squelettale est donc réinjecté en partie, de préférence en majeure partie, en amont de la zone de distillation afin de séparer l'isobutène formé des butènes linéaires. La quantité d'isobutène supplémentaire formé dans la zone d'isomérisation squelettale ne peut bien sûr être supérieure à la quantité de butènes linéaires déjà présent dans la charge initiale du procédé. Étant donné que l'effluent de la zone d'isomérisation squelettale du procédé selon l'invention est recyclé en partie, de préférence en majeure partie, en amont de la zone de distillation du procédé selon l'invention, le débit dans la zone d'isomérisation squelettale du procédé peut varier. Plus le débit au travers de ladite zone d'isomérisation squelettale du procédé est important, plus la quantité d'isobutène supplémentaire formé dans ladite zone d'isomérisation squelettale est important et donc plus le rendement total en isobutène est important. L'effluent de la zone d'isomérisation squelettale est en majeure partie recyclé en amont du procédé selon l'invention, en tant que charge partielle de la zone de distillation ou bien de préférence en tant que charge partielle de la première zone d'hydroisomérisation dans le cas préféré où celle-ci est présente.

La zone d'isomérisation squelettale fonctionne généralement dans les conditions opératoires données ci-après, bien connues de l'homme du métier, selon que le catalyseur comprend de la zéolithe ou non.

Lorsque le catalyseur d'isomérisation squelettale comprend de la zéolithe, la charge de la zone d'isomérisation squelettale est mise en contact avec le catalyseur d'isomérisation squelettale à une température comprise entre 150 et 500°C, de préférence entre 150 et 450 °C, et à une pression comprise entre 0,01 et 1, de préférence entre 0,01 et 0,5 MPa absolus. La vitesse spatiale est comprise entre 0,1 et 10, de préférence entre 0,5 et 6 h⁻¹, exprimée en volume de charge oléfinique par volume de catalyseur et par heure. Le catalyseur comprend alors généralement entre 5 et 100 %, de préférence entre 10 et 90 %, de façon encore plus préférée entre 20 et 80 % poids de tamis moléculaire (ou zéolithe) , le complément à 100 % poids étant assuré par la matrice. De préférence le tamis moléculaire est choisi dans le groupe formé par les tamis SAPO-31, SAPO-11, Théta-1, EU-1, Oméga, Mordénite, Ferriérite, Nu-10, Nu-86 et Nu-87, et la matrice est l'alumine.

Lorsque le catalyseur d'isomérisation squelettale ne comprend pas de la zéolithe, ledit catalyseur comprend principalement de l'alumine (généralement éta et/ou gamma) avec éventuellement au moins un additif tels que ceux à base de titane et/ou d'élément du groupe IIIA, ayant le plus souvent subi un prétraitement à la vapeur (voir par exemple les brevets US-A-3.558.733, US-A-2.417.647, US-A-4.013.590, US-A-4.038.337, US-A-4.434.315 ou US-A-5.321.195 ou les demandes de brevet GB-A-2.129.701 ou EP-A-0.696.265). Le procédé est effectué à une température comprise entre 300 et 570°C, de préférence entre 400 et 550°C, à une pression comprise entre 0,1 et 1 MPa, de préférence entre 0,1 et 0,5 MPa, à une vitesse spatiale comprise entre 0,1 et 10 h⁻¹, de préférece entre 0,5 et 6 h⁻¹, le rapport molaire eau injectée sur hydrocarbures oléfiniques étant compris entre 0,1 et 10, de préférence entre 0,5 et 3. Ledit procédé est par exemple décrit dans la demande de brevet EP-A-0.611.185.

Certains composés susceptibles d'être présents dans la charge étant inertes vis-à-vis des réactions mises en oeuvre dans le procédé et certains sous-produits pouvant être formé lors de l'une ou l'autre des étapes du procédé, étant donné que l'effluent de la zone d'isomérisation squelettale est recyclé en partie, de préférence en majeure partie, en amont de la zone de distillation, il est peut être nécessaire dans le cadre du procédé selon l'invention de prévoir au moins une purge afin d'éviter l'accumulation desdits composés dans le procédé. Par exemple, une purge peut être prévue en amont de la zone d'isomérisation squelettale du procédé afin d'éviter l'accumulation de normal butane s'il y en a dans la charge du procédé. Il est également possible de placer sur l'effluent de la zone d'isomérisation squelettale du procédé un moyen (par exemple une distillation) pour éliminer les composés plus légers que les hydrocarbures en C4 qui se seraient formés dans cette partie du procédé et un moyen (par exemple une distillation) pour éliminer les composés plus lourds que les hydrocarbures en C4 qui auraient pu se former dans cette partie du procédé.

### EXEMPLES

Des tests pilotes d'hydroisomérisation ont été effectués à partir d'une charge oléfinique C₄ sur un catalyseur d'hydroisomérisation LD267R, produit et commercialisé par la société Procatalyse, qui garnit chacun des lits catalytiques. Les résultats de ces tests sont présentés dans le tableau 1 ci-dessous ; ils ont permis de déterminer les paramètres de calcul qui permettent de simuler le procédé selon l'invention au moyen de logiciels adaptés. Le logiciel utilisé pour cette simulation est commercialisé sous le nom de Pro2 par la société SIMCI.

**Tableau 1 :**

| résultats de tests pilotes | | | | | | | | | |
|---|---|---|---|---|---|---|---|---|---|
| T°C | | 40 | 60 | 90 | 50 | 50 | 50 | 50 | 50 |
| VVH h-1 | | 30 | 30 | 30 | 30 | 30 | 30 | 30 | 30 |
| P bars | | 10 | 10 | 10 | 6,5 | 10 | 15 | 10 | 10 |
| H2/HC m/m | | 0,17 | 0,17 | 0,17 | 0,17 | 0,17 | 0,17 | 0,1 | 0,19 |
| | ch | eff | eff | eff | eff | eff | eff | eff | eff |
| < C4 | | 0,11 | 0,12 | 0,11 | 0,1 | 0,10 | 0,10 | 0,10 | 0,11 |
| iC4 | | 5,75 | 5,75 | 5,73 | 5,71 | 5,76 | 5,75 | 5,72 | 5,76 |
| iC4= | | 78,72 | 78,71 | 78,73 | 78,77 | 78,73 | 78,73 | 78,75 | 78,71 |
| 1-C4= | | 1,30 | 0,91 | 0,75 | 1,15 | 1,01 | 1,18 | 1,13 | 1,00 |
| n-C4 | | 7,19 | 7,17 | 7,14 | 7,14 | 7,18 | 7,19 | 7,16 | 7,20 |
| tr2-C4= | | 5,40 | 5,48 | 5,40 | 5,46 | 5,49 | 5,41 | 5,46 | 5,48 |
| cs2-C4= | | 1,54 | 1,85 | 2,14 | 1,66 | 1,74 | 1,64 | 1,69 | 1,75 |

où ch = charge et eff = effluent, et avec la légende suivante pour ce tableau et les tableaux suivants :

| | |
|---|---|
| < C₄ | composés à moins de 4 (4 exclu) atomes de carbone par molécule (ou C3⁻) |
| iC₄ | isobutane |
| i C₄⁼ | isobutène |
| 1-C₄⁼ | butène-1 |
| C₄⁼⁼ 1,3 | butadiène-1,3 |
| n C₄ | normal-butane |
| tr2-C₄⁼ | butène-2 trans |
| cs2-C₄⁼ | butène-2 cis |
| > C₄ | composés à plus de 4 (4 exclu) atomes de carbone par molécule (ou C5⁺) |

Deux exemples simulés par le calcul ont été effectués. Ils sont décrits ci-après. Dans ces deux exemples, la modélisation de la zone d'isomérisation squelettale a été faite de telle façon que l'on remette les butènes sensiblement à l'équilibre thermodynamique à la température de réaction, qui est généralement de 490 à 500°C. La distillation extractive est réalisée avec un solvant approprié pour permettre de recycler les butènes à extinction.

### Exemple 1 :

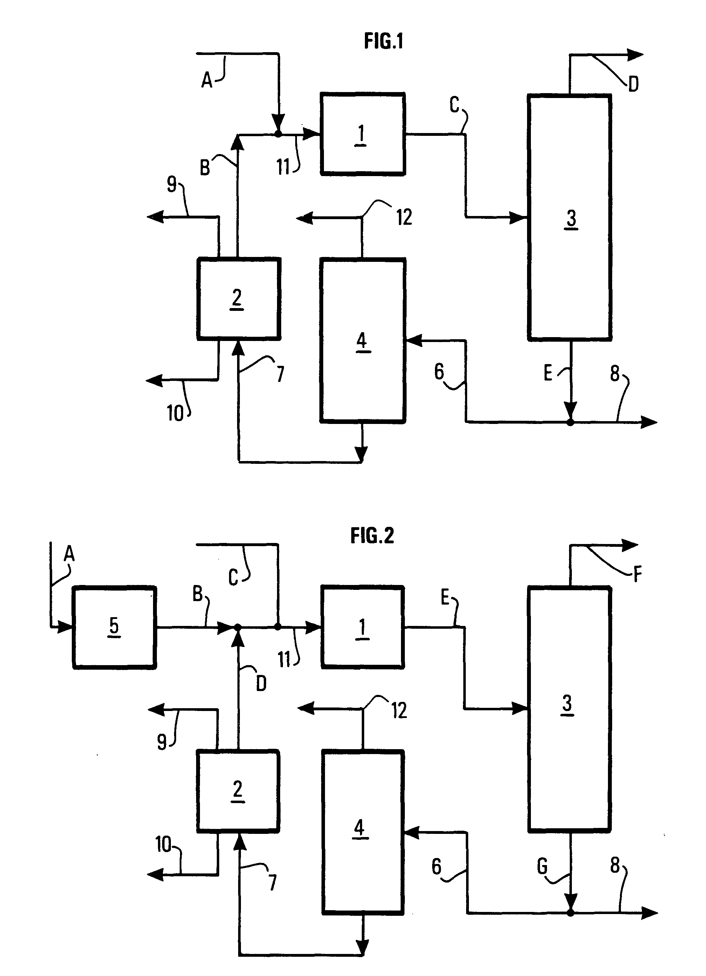

L'exemple 1 est conduit selon le shéma de procédé représenté figure 1. Le raffinat-1 est introduit par la ligne (A) puis par la ligne (11) dans la zone d'hydroisomérisation (1), en mélange avec l'effluent (B) de la zone d'isomérisation squelettale (2). L'effluent de la zone (1) est envoyé par la ligne (C) en zone de distillation réactive (3). La tête de la zone de distillation (D) comprend principalement de l'isobutène pur. Le fond de la zone de distillation (E) est en partie introduit par la ligne (6) dans la zone de distillation extractive (4), l'effluent (7) de ladite zone (4) servant de charge à la zone (2). Les purges éventuelles sont représentées par les lignes (8), (9), (10) et (12).

La configuration de l'unité de distillation réactive, comportant tois zones d'hydroisomérisation situées à l'extérieur de la colonne, est la suivante :
o colonne de 130 plateaux théoriques,
o plateau d'alimentation n° 90,
o les réacteurs extérieurs sont alimentés par des soutirages situés respectivement aux plateaux 10, 25 et 39. L'effluent de chacun des réacteurs extérieurs est réintroduit sur le plateau de soutirage alimentant le réacteur extérieur considéré.

### Réacteurs :

Les trois réacteurs contiennent chacun 10 tonnes de catalyseur.

Conditions opératoires :
taux de reflux : 20,
pression en tête de colonne : 6,2 bars absolu,
pression en fond de colonne : 7 bar absolu
température de l'alimentation de la colonne : 59 °C,
température en tête de colonne : 45 °C,
température en fond de colonne : 72 °C,
température du réacteur alimenté par un soutirage au plateau 10 : 53 °C
pression du réacteur alimenté par un soutirage au plateau 10 : 6,6 bar absolu
débit dans le réacteur alimenté par un soutirage au plateau 10 : 5200 kmole/h
température du réacteur alimenté par un soutirage au plateau 25 : 53,5 °C
pression du réacteur alimenté par un soutirage au plateau 25 : 6,6 bar absolu
débit dans le réacteur alimenté par un soutirage au plateau 25 : 5200 kmole/h
température du réacteur alimenté par un soutirage au plateau 39 : 54 °C
pression du réacteur alimenté par un soutirage au plateau 39 : 6,7 bar absolu
débit dans le réacteur alimenté par un soutirage au plateau 39 : 5200 kmole/h

Avec cette configuration et sous ces conditions opératoires, la simulation a conduit aux résultats suivants :

| | A (% poids) | B (% poids) | C (% poids) | D (% poids) | E (% poids) |
|---|---|---|---|---|---|
| < C₄ | 0,10 | 2,59 | 1,54 | 5,05 | - |
| i C₄ | 1,88 | 0 | 0,92 | 3,38 | - |
| i C₄= | 37,13 | 21,86 | 28,34 | 91,46 | 0,57 |
| 1-C₄⁼ | 27,72 | 12,09 | 7,31 | 0,04 | 0,38 |
| C₄==1,3 | 0,99 | - | - | - | - |
| n C4 | 16,93 | 1,59 | 8,85 | 0,02 | 0,73 |
| tr2-C₄= | 6,74 | 15,02 | 18,74 | 0,03 | 38,33 |
| cs2-C₄= | 8,42 | 12,09 | 14,18 | - | 27,06 |
| > C4 | 0,10 | 34,75 | 20,12 | - | 32,94 |
| Total (kt/an) | 150 | 205 | 355 | 108 | 217 |

A : Charge de la première unité d'hydroisomérisation,
B : Effluent de l'unité d'isomérisation squelettale mélangé au flux A en amont de la première unité d'hydroisomérisation,
C : Effluent de la première unité d'hydroisomérisation ; ce flux est également la charge de la distillation réactive,
D : Effluent de tête de la distillation réactive,
E : Effluent de fond de la distillation réactive.

Rendement total en isobutène par rapport à l'isobutène de la charge : 177 % Rendement en isobutène par rapport au butènes iso et normaux et au butadiène :
82 %
Butène-1 dans isobutène en tête de colonne : 4,5.10⁻⁴.

### Exemple 2:

L'exemple 2 est conduit selon le schéma de procédé représenté figure 2. La charge C₄ issue d'un vapocraquage à la vapeur est introduite par la ligne (A) dans une zone d'hydrogénation (5). L'effluent de la zone (5) est introduit par la ligne (B) puis par la ligne (11) dans la zone d'hydroisomérisation (1), en mélange avec la raffinat-1 de la ligne (C) et avec l'effluent (D) de la zone d'isomérisation squelettale (2). L'effluent de la zone (1) est envoyé par la ligne (E) en zone de distillation réactive (3). La tête de la zone de distillation (F) comprend principalement de l'isobutène pur. Le fond de la zone de distillation (G) est en partie introduit par la ligne (6) dans la zone de distillation extractive (4), l'effluent (7) de ladite zone (4) servant de charge à la zone (2). Les purges éventuelles sont représentées par les lignes (8), (9), (10) et (12).

La configuration de l'unité de distillation réactive, comportant trois zones d'hydroisomérisation situées à l'extérieur de la colonne, est la suivante :
o colonne de 130 plateaux théoriques,
o plateau d'alimentation n° 90,
o les réacteurs extérieurs sont alimentés par des soutirages situés respectivement aux plateaux 10, 25 et 39. L'effluent de chacun des réacteurs extérieurs est réintroduit sur le plateau de soutirage alimentant le réacteur extérieur considéré.

### Réacteurs :

Les trois réacteurs contiennent chacun 10 tonnes de catalyseur.

Conditions opératoires :
taux de reflux : 20,
pression en tête de colonne : 6,2 bars absolu,
pression en fond de colonne : 7 bar absolu
température de l'alimentation de la colonne : 59 °C,
température en tête de colonne : 44 °C,
température en fond de colonne : 72,5 °C,
température du réacteur alimenté par un soutirage au plateau 10 : 53 °C
pression du réacteur alimenté par un soutirage au plateau 10 : 6,6 bar absolu
débit dans le réacteur alimenté par un soutirage au plateau 10 : 5200 kmole/h
température du réacteur alimenté par un soutirage au plateau 25 : 53,5 °C
pression du réacteur alimenté par un soutirage au plateau 25 : 6,6 bar absolu
débit dans le réacteur alimenté par un soutirage au plateau 25 : 5200 kmole/h
température du réacteur alimenté par un soutirage au plateau 39 : 54 °C
pression du réacteur alimenté par un soutirage au plateau 39 : 6,7 bar absolu
débit dans le réacteur alimenté par un soutirage au plateau 39 : 5200 kmole/h

Avec cette configuration et sous ces conditions opératoires, la simulation a conduit aux résultats suivants :

| | A (% poids) | B (% poids) | C (% poids) | D (% poids) | E (% poids) | F (% poids) | G (% poids) |
|---|---|---|---|---|---|---|---|
| < C₄ | 0,05 | 0,05 | 0,10 | 2,61 | 1,71 | 6,59 | 0,00 |
| i C₄ | 0,95 | 1,95 | 1,88 | 0,00 | 0,77 | 3,37 | 0,00 |
| i C₄= | 18,74 | 17,74 | 37,13 | 22,05 | 23,86 | 89,99 | 0,54 |
| 1-C₄⁼ | 13,99 | 28,99 | 27,72 | 12,20 | 2,91 | 0,02 | 0,16 |
| C₄⁼⁼ 1,3 | 50,01 | 1,00 | 0,99 | 0,00 | 0,00 | 0,00 | 0,00 |
| n C₄ | 8,55 | 9,56 | 16,93 | 1,31 | 6,05 | 0,01 | 0,44 |
| tr2-C₄⁼ | 3,40 | 27,41 | 6,74 | 15,15 | 25,73 | 0,01 | 40,40 |
| cs2-C₄⁼ | 4,25 | 13,25 | 8,42 | 12,20 | 16,68 | 0,00 | 25,80 |
| > C₄ | 0,05 | 0,05 | 0,10 | 34,47 | 22,29 | 0,00 | 32,65 |
| Total (kt/an) | 75 | 75 | 75 | 273 | 423 | 110 | 273 |

A : Alimentation de l'unité d'hydrogénation sélective du butadiène,
B : Effluent de l'unité d'hydrogénation sélective du butadiène,
C : Complément de charge mélangée au flux B et au flux D en amont de la première unité d'hydroisomérisation(cette charge est du type raffinat-1),
D : Effluent de l'unité d'isomérisation squelettale mélangée aux flux B et C en amont de la première unité d'hydroisomérisation,
E : Effluent de la première unité d'hydroisomérisation sélective ; ce flux est également la charge de la distillation réactive,
F : Effluent de tête de la distillation réactive,
G : Effluent de fond de la distillation réactive.

Rendement total en isobutène par rapport à l'isobutène de la charge : 236 % Rendement en isobutène par rapport au butènes iso et normaux et au butadiène : 78 % Butène-1 dans isobutène en tête de colonne : 2.10⁻⁴.

Ainsi les deux exemples de réalisation du procédé selon l'invention ont permis de montrer la mise en valeur de la combinaison des différentes zones de réaction et de distillation, qui a conduit un rendement exceptionnel en isobutène pur produit par rapport à l'isobutène présent dans la charge de la première unité d'hydroisomérisation.

## Revendications

1. Procédé de production d'isobutène comportant au moins :
a) le traitement d'une charge, comprenant en majeure partie des hydrocarbures oléfiniques comportant 4 atomes de carbone par molécule dont de l'isobutène, ainsi que du butène-1 et des butènes-2 en rapport correspondant sensiblement à l'équilibre thermodynamique, dans une zone de distillation réactive comprenant une zone de distillation associée à une zone réactionnelle d'hydroisomérisation,
b) le soutirage en tête de ladite zone de distillation réactive d'un effluent riche en isobutène et en fond de ladite zone de distillation réactive d'un effluent riche en butènes-2,
c) le passage de la majeure partie dudit effluent riche en butènes-2 dans une seconde zone de distillation de manière à obtenir un premier effluent comprenant en majeure partie des butènes-2 et un second effluent comprenant en majeure partie du normal butane,
d) l'introduction, en majeure partie, dudit premier effluent, dans une zone d'isomérisation squelettale et
e) le recyclage d'une partie de l'effluent de la zone d'isomérisation squelettale en amont de la zone de distillation réactive.

2. Procédé selon la revendication 1 tel que la majeure partie de l'effluent de la zone d'isomérisation squelettale est recyclée en amont de la zone de distillation.

3. Procédé selon l'une des revendications 1 ou 2 tel que la charge est obtenue par traitement d'une coupe comprenant en majeure partie des hydrocarbures oléfiniques comportant 4 atomes de carbone par molécule, dans une première zone d'hydroisomérisation, la majeure partie de l'effluent de ladite première zone d'hydroisomérisation servant alors de charge qui alimente la zone de distillation.

4. Procédé selon la revendication 3 tel que le recyclage de la partie de l'effluent de la zone d'isomérisation squelettale est réalisé en amont de la première zone d'hydroisomérisation.

5. Procédé selon l'une des revendications 1 à 4 tel que la zone d'hydroisomérisation associée à la zone de distillation est totalement interne à ladite zone de distillation.

6. Procédé selon l'une des revendications 1 à 4 tel que la zone d'hydroisomérisation associée à la zone de distillation est totalement externe à ladite zone de distillation.

7. Procédé selon l'une des revendications 1 à 4 tel que la zone d'hydroisomérisation associée à la zone de distillation est à la fois interne et externe à ladite zone de distillation.

8. Procédé selon l'une des revendications 1 à 7 tel que toute zone d'hydroisomérisation comprise dans le procédé selon l'invention est telle que la réaction d'hydroisomérisation est réalisée en présence d'un catalyseur d'hydroisomérisation et d'un flux gazeux comprenant de l'hydrogène.

9. Procédé selon la revendication 8 tel que le catalyseur d'hydroisomérisation est un catalyseur supporté comprenant au moins un métal noble du groupe VIII choisi dans le groupe formé par le ruthénium, le rhodium, le palladium, l'osmium, l'iridium et le platine, ou le nickel, traité par un composé contenant du soufre puis par de l'hydrogène avant son utilisation.

10. Procédé selon l'une des revendications 1 à 9 tel que la seconde zone de distillation est une zone de distillation extractive.

## Patentansprüche

1. Verfahren zur Herstellung von Isobuten, das wenigstens umfasst:
a) die Behandlung einer Charge, die zum größeren Teil olefinische Kohlenwasserstoffe mit 4 Kohlenstoffatomen pro Molekül, darunter Isobuten sowie 1-Buten und 2-Butene in einem im wesentlichen dem thermodynamischen Gleichgewicht entsprechenden Verhältnis umfasst, in einer reaktiven Destillationszone, die eine Destillationszone umfasst, die einer Hydroisomerisierungsreaktionszone zugeordnet ist,
b) das Abziehen eines an Isobuten reichen Abstroms am Kopf der reaktiven Destillationszone und eines an 2-Butenen reichen Abstroms am Boden der reaktiven Destillationszone,
c) den Durchgang des größeren Teils dieses an 2-Butenen reichen Abstroms in eine zweite Destillationszone derart, dass ein erster zum größeren Teil 2-Butene umfassender Abstrom und eine zweiter zum größeren Teil Normalbutan umfassender Abstrom erhalten wird,
d) die Einführung, zum größeren Teil des ersten Abstroms in eine Gerüstisomerisierungszone und
e) das Rezyklieren eines Teils des Abstroms der Gerüstisomerisierungszone vor der reaktiven Destillationszone.

2. Verfahren nach Anspruch 1 derart, dass der größere Teil des Abstroms der Gerüstisomerisierungszone vor der Destillationszone rezykliert wird.

3. Verfahren nach einem der Ansprüche 1 oder 2 derart, dass die Charge durch Behandlung eines Schnittes erhalten wird, der zum größeren Teil olefinische Kohlenwasserstoffe mit 4 Kohlenstoffatomen pro Molekül umfasst, in einer ersten Hydroisomerisierungszone, wobei der größere Teil des Abstroms der zweiten Hydroisomerisierungszone so als Charge dient, die die Destillationszone speist.

4. Verfahren nach Anspruch 3 derart, dass die Rezyklierung des Teils des Abstroms der Gerüstisomerisierungszone vor der ersten Hydroisomerisierungszone verwirklicht wird.

5. Verfahren nach einem der Ansprüche 1 bis 4 derart, dass die der Destillationszone zugeordnete Hydroisomerisierungszone vollständig innerhalb der Destillationszone liegt.

6. Verfahren nach einem der Ansprüche 1 bis 4 derart, dass die der Destillationszone zugeordnete Hydroisomerisierungszone vollständig außerhalb der Destillationszone liegt.

7. Verfahren nach einem der Ansprüche 1 bis 4 derart, dass die der Destillationszone zugeordnete Hydroisomerisierungszone gleichzeitig innerhalb und außerhalb der Destillationszone liegt.

8. Verfahren nach einem der Ansprüche 1 bis 7 derart, dass jede Hydroisomerisierungszone, die in dem Verfahren gemäß der Erfindung enthalten ist, derart ist, dass die Hydroisomerisierungsreaktion in Gegenwart von einem Hydroisomerisierungskatalysator und einem gasförmigen Wasserstoff umfassenden Strom durchgeführt wird.

9. Verfahren nach Anspruch 8 derart, dass der Hydroisomerisierungskatalysator ein Katalysator mit Träger ist, der wenigstens ein Edelmetall der Gruppe VIII umfasst, das aus der Gruppe gewählt ist, die gebildet wird durch Ruthenium, Rhodium, Palladium, Osmium, Iridium und Platin oder Nickel, und zwar vor dessen Verwendung behandelt durch eine schwefelhaltige Verbindung und dann durch Wasserstoff.

10. Verfahren nach einem der Ansprüche 1 bis 9 derart, dass die zweite Destillationszone eine extraktive Destillationszone ist.

## Claims

1. A process for the production of isobutene comprising at least:
a) treating a feed, the major portion comprising olefinic hydrocarbons containing 4 carbon atoms per molecule, including isobutene, also butene-1 and butene-2 compounds in a ratio which substantially corresponds to the thermodynamic equilibrium, said feed being treated in a reactive distillation zone comprising a distillation zone associated with a hydroisomerisation reaction zone,
b) producing an overhead effluent from the reactive distillation zone which is rich in isobutene, and a bottom effluent from the reactive distillation zone which is rich in butene-2 compounds,
c) passing the major portion of said bottom effluent rich in butene-2 compounds to a second distillation zone, so as to produce a first effluent comprising butene-2 compounds as its major portion and a second effluent comprising normal-butane as its major portion,
d) introducing the major portion of said first effluent into a skeletal isomerisation zone, and
e) recycling a portion of the effluent from the skeletal isomerisation zone upstream of the reactive distillation zone

2. A process according to claim 1, in which the major portion of the effluent from the skeletal isomerisation zone is recycled upstream of the distillation zone.

3. A process according to claim 1 or claim 2, in which the feed is obtained by treating, in a first hydroisomerisation zone, a cut comprising as its major portion olefinic hydrocarbons containing 4 carbon atoms per molecule, the major portion of the effluent from said first hydroisomerisation zone then acting as the feed which is supplied to the distillation zone.

4. A process according to claim 3, in which said portion of effluent from the skeletal isomerisation zone is recycled upstream of the first hydroisomerisation zone.

5. A process according to any one of claims 1 to 4, in which the hydroisomerisation zone associated with the distillation zone is completely internal to said distillation zone.

6. A process according to any one of claims 1 to 4, in which the hydroisomerisation zone associated with the distillation zone is completely external of said distillation zone.

7. A process according to any one of claims 1 to 4, in which the hydroisomerisation zone associated with the distillation zone is both internal to and external of said distillation zone.

8. A process according to any one of claims 1 to 7, in which each hydroisomerisation zone in the process of the invention is such that he hydroisomerisation reaction is carried out in the presence of a hydroisomerisation catalyst and a gaseous stream containing hydrogen.

9. A process according to claim 8, in which the hydroisomerisation catalyst is a supported catalyst comprising at least one noble metal from group VIII selected from the group formed by ruthenium, rhodium, palladium, osmium, iridium and platinum, or nickel, treated by a compound containing sulphur then by hydrogen before its use.

10. A process according to any one of claims 1 to 9, in which the second distillation zone is an extractive distillation zone.
